# FASCICULE DE BREVET EUROPEEN

(11) **EP 0 717 785 B1**
(45) Date de publication et mention de la délivrance du brevet: **30.07.1997**
(21) Numéro de dépôt: 94926284.4
(22) Date de dépôt: 07.09.1994
(51) Int. Cl.: C23C 14/04, C23C 16/04, C23C 14/28

(54) **DISPOSITIF DE TRAITEMENT D'UN MATERIAU A TETE PHOTO-IONIQUE MINIATURISEE**
VORRICHTUNG ZUR MATERIALBEARBEITUNG MIT MINIATURISIERTEM PHOTOIONISCHEM KOPF
DEVICE WITH MINIATURIZED PHOTOIONIC HEAD FOR THE TREATMENT OF A MATERIAL

(30) Priorité: 08.09.1993 FR 9310657
(43) Date de publication de la demande: 26.06.1996
(73) Titulaire: COMMISSARIAT A L'ENERGIE ATOMIQUE, 75015 Paris (FR)
(72) Inventeur: ELOY, Jean-François Résidence Rosier-Bellevue A2, F-33170 Gradignan (FR)
(74) Mandataire: Dubois-Chabert, Guy
(86) Numéro de dépôt international: FR9401054
(87) Numéro de publication internationale: WO9507368

(56) Documents cités:
- EP-A- 0 239 432
- EP-A- 0 546 921
- DE-A- 2 523 982
- FR-A- 2 083 740
- FR-A- 2 623 820
- US-A- 4 752 455
- US-A- 4 778 693

## Description

La présente invention concerne un dispositif de traitement d'un matériau, à tête photo-ionique miniaturisée.

Elle permet notamment de réaliser, sur une grande variété de surfaces d'objets de petites dimensions et très difficiles d'accès, les traitements micro-localisés suivant :
- placages,
- colmatages de fissures,
- durcissements superficiels,
- fabrication de nouveaux alliages,
- fabrication de super-alliages.

Elle s'applique notamment :
- en chirurgie dentaire, au traitement localisé des dents (fissures, caries et prothèses in situ sur un patient), pour aboutir à un renforcement de l'émail et de la résistance à la carie,
- à la micro-électronique (dépôt localisé de couches spécifiques, restauration ou anihilation de fonctions des composants d'un micro-circuit, réalisation de nouveaux types d'alliages pour connexions électriques),
- à la micro-mécanique (durcissement superficiel d'alliages peu coûteux pour l'horlogerie),
- au traitement automatique localisé en atmosphère chimiquement agressive ou radioactive,
- au traitement automatique localisé en milieu liquide, en particulier sous-marin.

On sait que l'implantation ionique de certaines espèces chimiques dans la couche superficielle de certains alliages, modifie les propriétés mécaniques superficielles de ceux-ci (résistance à l'usure, au frottement, à l'abrasion sableuse, à la corrosion par exemple).

De plus, il est connu que, parmi les effets induits par l'impact d'un faisceau laser sur un matériau solide, figure l'apparition d'une vapeur ionisée et fréquemment d'un plasma qui, par leurs effets de détente, induisent une onde de recul qui, elle-même, peut entraîner un phénomène de trempe superficielle sur le matériau dont cette vapeur et ce plasma sont issus.

Il est également connu que l'impact d'un faisceau laser sur une couche superficielle déposée sur un support transparent peut induire, par transmission du faisceau laser à travers ce support, l'évaporation de la couche déposée qui peut être, de ce fait, consécutivement condensée sur un autre support.

On sait aussi que le dépôt d'une couche mince par pulvérisation cathodique peut être assisté par l'impact d'un faisceau laser sur la couche mince en cours de condensation et de croissance.

Ceci a pour conséquence l'amélioration des qualités de la couche déposée (cohésion, adhérence, propriétés diélectriques).

On consultera à ce sujet le document suivant :
(1) Demande de brevet français n° 8306749 du 25 avril 1983, "Procédé de croissance amorphe d'un corps avec cristallisation sous rayonnement", André Chenevas-Paule, Robert Cuchet et Jean-François Eloy (FR-A-2 544 752) - voir aussi EP-A-0127499 et US-A-4529617.

Il est également connu que la combinaison ou le couplage de plusieurs effets (tel que l'évaporation explosive, l'implantation ionique induite par une différence de potentiel électrique de séparation des charges électroniques et ioniques, l'onde thermique induite par la condensation du plasma ionique, la trempe ultra-rapide induite par le puits thermique représenté par la capacité thermique du matériau recevant ce plasma ionique) rendus simultanés par l'usage d'un faisceau laser pulsé de grande puissance, peut engendrer des couches superficielles de matériau ayant de nouvelles propriétés mécaniques et physico-chimiques propres à améliorer leur utilisation (résistance à l'usure, au frottement, à l'abrasion sableuse, à la corrosion ...).

On consultera à ce sujet le document suivant :
(2) Demande de brevet français n°8602568 du 25 février 1986, "Procédé et dispositif de traitement d'un matériau par effet thermo-ionique en vue d'en modifier ses propriétés physico-chimiques", Jean-François Eloy (FR-A-2594853) - voir aussi EP-A-0239432 et US-A-4714628.

Les dispositifs connus de traitement de surface par onde de choc ne permettent pas de mettre en oeuvre les différents processus et phénomènes décrits ci-dessus pour toutes les formes de surfaces ou de pièces à traiter.

En effet, ces dispositifs connus sont encombrants et, en ce qui concerne en particulier le dispositif décrit dans le document (2), la propagation rectiligne du faisceau laser utilisé dans ce dispositif nécessite :
- une vision directe de la surface à traiter et un accès direct du faisceau laser à cette surface à traiter,
- une fenêtre transparente jouant d'une part le rôle de source matérielle fixe et rigide pour l'onde de choc et d'autre part le rôle de surface de référence fixant la direction de propagation de cette onde de choc (qui est induite par l'évaporation due au laser) vers le matériau à traiter,
- une bague électriquement isolante permettant le confinement de l'onde de pression du plasma ionisé de manière à la guider en direction de la surface à traiter, cette bague isolante servant aussi d'entretoise d'écartement séparant la couche à faire exploser de la surface à traiter.

Ces différents éléments du dispositif décrit dans le document (2) limitent l'application de ce dispositif à des surfaces planes ou éventuellement légèrement incurvées.

De plus, les caractéristiques géométriques du faisceau laser utilisé dans ce dispositif limitent les traitements spécifiques, que l'on peut faire subir grâce à ce dispositif, à de larges surfaces auxquelles on peut accéder relativement facilement et directement.

Pour d'autres surfaces, ces caractéristiques du faisceau laser nécessitent la mise au point de systèmes optiques sophistiqués et spécifiques à chaque forme géométrique de pièce à traiter.

Le dispositif décrit dans le document (2) ne permet pas le traitement de surfaces de formes quelconques et surtout de petites surfaces qui sont localisées en des zones d'une pièce ou d'un dispositif de forme complexe, qui réservent à leur traitement un espace restreint et auxquelles on ne peut accéder de façon rectiligne.

La présente invention a pour but de remédier aux inconvénients précédents, en proposant un dispositif de traitement d'un matériau à l'aide d'un faisceau lumineux intense et d'au moins une fibre optique pour transporter celui-ci.

Cette fibre optique, munie d'une tête photo-ionique miniaturisée (c'est-à-dire dont les dimensions transversales ne dépassent que légèrement, d'environ 20%, celles de la fibre optique), permet à ce faisceau d'être guidé et d'accéder à toute surface, de dimension réduite et d'accès géométriquement restreint, d'une pièce de structure et de forme quelconques.

En outre, dans certains modes de réalisation particuliers, le dispositif objet de l'invention permet une mise en place, simplifiée et programmable en position dans l'espace, d'une ou plusieurs têtes photo-ioniques miniaturisées, en vue d'un traitement, en particulier d'une implantation ionique, assisté par laser.

De façon précise, la présente invention a pour objet un dispositif de traitement d'un matériau, caractérisé en ce qu'il comprend :
- au moins une fibre optique,
- une première source de lumière prévue pour engendrer une lumière pulsée permettant le traitement du matériau,
- un moyen d'injection de cette lumière pulsée dans une première extrémité de chaque fibre optique, et
   - pour chaque fibre optique, une tête photo-ionique miniaturisée comprenant une bague de confinement dont un premier côté est rendu solidaire de la deuxième extrémité de la fibre tandis que le deuxième côté de la bague est destiné à être placé contre le matériau, cette bague étant apte à confiner un plasma susceptible de se former, grâce à la lumière pulsée, dans l'espace délimité par cette bague, à partir du matériau ou d'une matière qui est placée dans cet espace, en vue du traitement du matériau.

La ou les têtes photo-ioniques miniaturisées du dispositif objet de l'invention permettent, comme le dispositif connu, décrit dans le document (2), un durcissement superficiel localisé d'un matériau, mais le dispositif objet de l'invention est d'un encombrement incomparablement plus faible que ce dispositif connu, du fait qu'il emploie une ou des têtes photo-ioniques miniaturisées.

Selon un mode de réalisation particulier du dispositif objet de l'invention, la bague de confinement est électriquement isolante, chaque fibre optique est revêtue d'une gaine électriquement conductrice, la matière placée dans l'espace délimité par la bague de confinement forme une couche mince solide qui est placée contre la deuxième extrémité de la fibre optique et se trouve à distance du matériau, et qui est destinée à se transformer, grâce à la lumière pulsée, en un plasma contenant des ions, et le dispositif comprend en outre un moyen de polarisation de la gaine électriquement conductrice par rapport au matériau pour créer, dans l'espace délimité par la bague de confinement, un champ électrique apte à accélérer les ions du plasma vers le matériau en vue de les implanter dans celui-ci.

La matière formant la couche mince solide peut être électriquement conductrice ou semiconductrice.

Cette matière peut aussi être électriquement isolante et dans ce cas le dispositif objet de l'invention comprend en outre une bague qui est électriquement conductrice et reliée à la gaine conductrice et qui est placée sensiblement au niveau de la couche mince, contre la paroi externe de la bague de confinement.

Dans une réalisation particulière, le dispositif objet de l'invention comprend en outre une lentille qui est optiquement couplée à la deuxième extrémité de la fibre optique et qui comporte une face convexe dans l'espace délimité par la bague de confinement, cette face étant destinée à se trouver du côté du matériau à traiter, et la couche mince est formée sur cette face convexe.

Dans une autre réalisation particulière, la deuxième extrémité de la fibre optique comporte une partie convexe dans l'espace délimité par la bague de confinement et la couche mince est formée sur cette partie convexe.

Dans une autre réalisation particulière, le dispositif objet de l'invention comprend en outre une pastille qui est transparente à la lumière pulsée, qui est optiquement couplée à la deuxième extrémité de la fibre optique et placée dans l'espace délimité par la bague de confinement et dont la face destinée à se trouver du côté du matériau à traiter porte la couche mince.

Dans une autre réalisation particulière, le dispositif comprend en outre :
- une bande sur laquelle est formée la couche mince, et
- un moyen de défilement de cette bande entre la deuxième extrémité de la fibre optique et la bague de confinement.

Le dispositif objet de l'invention peut comprendre en outre une couche intermédiaire entre la fibre et la couche mince, cette couche intermédiaire étant prévue pour adapter l'indice optique de la fibre à celui de la couche mince, pour réduire la quantité de lumière pulsée susceptible d'être réfléchie par cette couche mince.

Selon un autre mode de réalisation particulier, le dispositif objet de l'invention comprend en outre, dans l'espace délimité par la bague de confinement, un fluide qui remplit cet espace et qui est transparent à la lumière pulsée, de façon à durcir le matériau par confinement du plasma formé par interaction de la lumière pulsée avec ce matériau, ou qui est photodécomposable et photoionisable par cette lumière pulsée, de façon à traiter chimiquement la surface du matériau par les espèces chimiques produites lors du processus de photoionisation et photodécomposition.

La première source de lumière peut être un laser de puissance pulsé.

En variante, la première source de lumière est une source de puissance, de lumière incohérente pulsée, et le dispositif comprend en outre une diode-laser qui est optiquement couplée à la deuxième extrémité de la fibre optique et qui est apte à engendrer un faisceau laser pulsé de puissance lorsqu'elle reçoit la lumière pulsée de la première source.

Dans une réalisation particulière du dispositif objet de l'invention, la fibre optique comprend :
- une fibre optique centrale, destinée à transmettre la lumière pulsée, et
- une fibre optique annulaire qui entoure cette fibre optique centrale,
et le dispositif comprend en outre :
- une deuxième source de lumière prévue pour engendrer une lumière visible permettant l'observation du matériau,
- un moyen d'injection de cette lumière d'observation dans la fibre optique annulaire, et
- un moyen de visualisation de l'image du matériau, à partir de la lumière d'observation réfléchie par le matériau et transmise par la fibre optique annulaire.

Le dispositif objet de l'invention peut comprendre en outre un moyen de positionnement de la tête optique en regard d'une zone choisie du matériau.

Le dispositif objet de l'invention peut comprendre une pluralité de fibres optiques respectivement associées à une pluralité de têtes photo-ioniques, pour traiter simultanément une pluralité de zones du matériau.

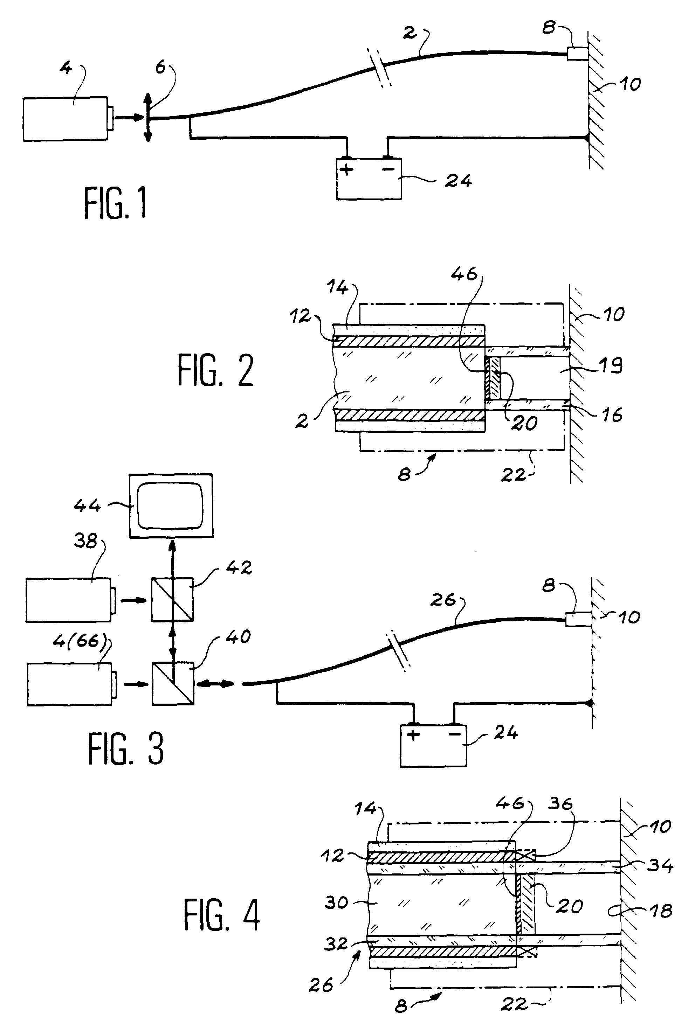

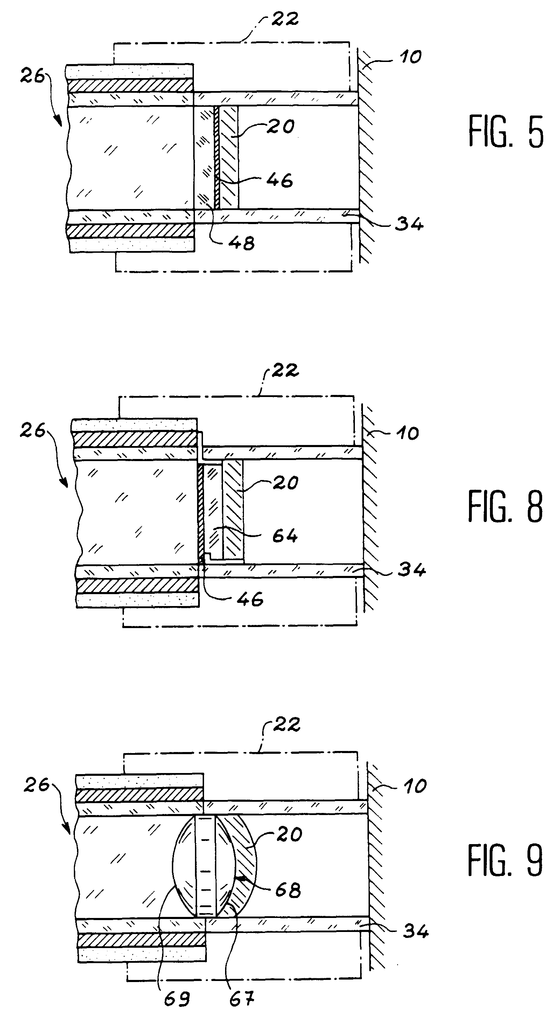

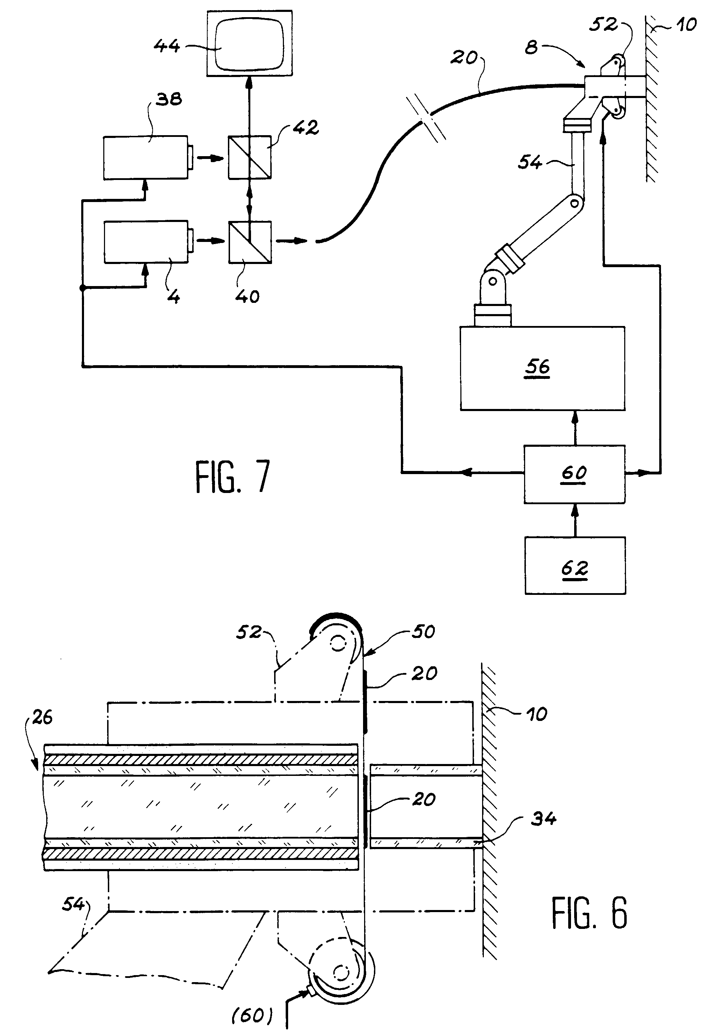

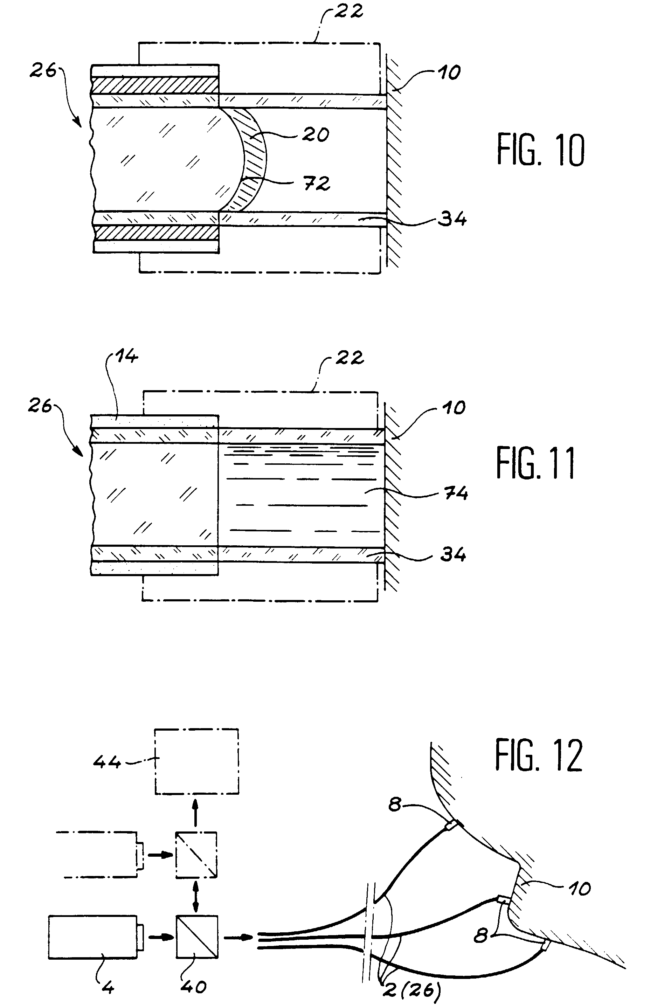

La présente invention sera mieux comprise à la lecture de la description d'exemples de réalisation donnés ci-après à titre purement indicatif et nullement limitatif, en faisant référence aux dessins annexés sur lesquels :
◆ la figure 1 est une vue schématique d'un mode de réalisation particulier du dispositif objet de l'invention,
◆ la figure 2 est une vue schématique de la tête photo-ionique miniaturisée que comporte le dispositif de la figure 1,
◆ la figure 3 est une vue schématique d'un autre mode de réalisation particulier du dispositif objet de l'invention, muni de moyens d'observation du matériau à traiter,
◆ la figure 4 est une vue schématique de la tête photo-ionique miniaturisée que comporte le dispositif de la figure 3,
◆ la figure 5 est une vue schématique de la tête photo-ionique d'un dispositif conforme à l'invention, dans lequel une couche mince à projeter sur un matériau est montée sur une pastille transparente au faisceau laser de puissance utilisé avec ce dispositif,
◆ la figure 6 est une vue schématique et partielle d'un autre dispositif conforme à l'invention utilisable de façon continue,
◆ la figure 7 illustre schématiquement la possibilité de robotiser un dispositif conforme à l'invention,
◆ la figure 8 est une vue schématique de la tête photo-ionique d'un autre dispositif conforme à l'invention, dans lequel une couche mince à projeter sur un matériau est montée sur une diode laser,
◆ la figure 9 est une vue schématique de la tête photo-ionique d'un autre dispositif conforme à l'invention, dans lequel la couche mince à projeter est montée sur une lentille,
◆ la figure 10 est une vue schématique de la tête photo-ionique d'un autre dispositif conforme à l'invention, dans lequel la couche mince à projeter est montée sur l'extrémité convexe d'une fibre optique,
◆ la figure 11 est une vue schématique de la tête photo-ionique d'un autre dispositif conforme à l'invention, dans lequel un fluide remplit l'espace délimité par une bague de confinement que comporte le dispositif, et
◆ la figure 12 illustre schématiquement un dispositif conforme à l'invention, comportant plusieurs têtes photo-ioniques.

Le dispositif de traitement conforme à l'invention, qui est schématiquement représenté sur la figure 1, comprend :
- une fibre optique 2,
- un laser de puissance 4 apte à émettre un faisceau laser pulsé,
- un moyen 6 d'injection de ce faisceau laser dans l'une des deux extrémités de la fibre optique 2, et
- une tête photo-ionique miniaturisée 8 qui est montée à l'autre extrémité de la fibre 2.

Cette tête photo-ionique miniaturisée 8, qui permet le traitement d'un matériau 10, est schématiquement représentée sur la figure 2.

On n'a pas représenté le coeur ni la gaine optique de la fibre 2.

La face de l'extrémité de cette fibre, en laquelle est montée la tête 8, est plane.

On voit que cette fibre 2 comprend une gaine électriquement conductrice 12 (par exemple une gaine métallique tressée) qui entoure la gaine optique de la fibre sur toute la longueur de celle-ci, ainsi qu'une autre gaine électriquement isolante 14 qui entoure la gaine 12 et qui joue le rôle de gaine de protection.

La tête 8 comprend une bague électriquement isolante 16 dont le diamètre extérieur est sensiblement égal au diamètre extérieur de la gaine optique de la fibre et qui est placée, d'un côté, contre la face plane de l'extrémité de cette fibre tandis que l'autre côté de la bague 16 est destiné à venir contre le matériau à traiter 10.

Plus précisément, la tête 8 est positionnée de façon que la bague 16 entoure une zone 18 du matériau que l'on veut traiter.

Cette tête 8 comprend en outre une couche mince 20 faite d'une matière que l'on veut projeter, comme on le verra par la suite, par une onde de choc, en direction de la zone 18 à traiter.

Cette couche mince 20 est placée dans l'espace délimité par la bague 16, contre la face plane de l'extrémité de la fibre 2, et se trouve ainsi à distance de la zone à traiter 18.

Une pièce 22 électriquement isolante, dont les dimensions transversales (comptées perpendiculairement à l'axe de l'extrémité de la fibre optique) ne dépassent que légèrement, d'environ 20%, le diamètre de la gaine 14, est destinée à rendre rigidement solidaires l'une de l'autre la bague 16 contenant la couche mince 20 et l'extrémité de la fibre 2.

Le dispositif des figures 1 et 2 comprend également un moyen 24 permettant de polariser positivement, de façon constante au cours du temps, la gaine conductrice 12 par rapport au matériau à traiter 10, ce dernier étant mis à la masse.

Cette polarisation est choisie de façon à pouvoir établir, dans l'espace délimité par la gaine 16, un champ électrique capable d'accélérer des ions formés à partir de la couche mince 20, comme on le verra par la suite, en vue d'implanter ces ions dans la zone 18 du matériau 10.

Pour implanter les ions dans la zone 18 du matériau, en face de laquelle se trouve la couche mince 20, on procède de la façon suivante.

On déclenche le laser 4 qui émet alors une impulsion lumineuse de puissance et cette impulsion est injectée dans la fibre 2 et s'y propage jusqu'à la tête photo-ionique 8.

Cette impulsion de puissance atteint la couche mince 20 et provoque l'évaporation de celle-ci de manière explosive.

Un plasma se forme dans l'espace 19 délimité par la bague 16 et les ions (positifs) de ce plasma sont accélérés en direction de la zone 18 grâce au champ électrique établi comme on l'a indiqué plus haut.

Ils sont alors implantés dans cette zone 18 (tandis que les électrons du plasma sont accélérés en sens contraire vers la fibre 2).

La bague 16 sert à confiner le plasma et l'onde explosive engendrée lors de la formation de celui-ci, à l'espace 19 et donc à la zone 18.

Le dispositif conforme à l'invention, qui est schématiquement représenté sur la figure 3, permet l'observation de la zone que l'on veut traiter et donc un positionnement correct de la tête photo-ionique en regard de cette zone puis, après le traitement de cette zone, permet une observation de la zone traitée.

Le dispositif de la figure 3 comprend une fibre optique 26 dont une extrémité est munie d'une tête photo-ionique encore référencée 8.

La fibre 26 est une fibre optique double, comprenant une fibre optique centrale 30 et une fibre optique annulaire 32 qui entoure la fibre 30, comme on le voit sur la figure 4.

La fibre 26 comprend encore la gaine conductrice 12 qui, dans le cas présent, entoure la fibre annulaire 32, ainsi que la gaine protectrice électriquement isolante 14 qui entoure encore la gaine 12.

Dans le dispositif de la figure 3, la tête photo-ionique comprend encore une bague électriquement isolante 34 dont le diamètre intérieur et le diamètre extérieur correspondent sensiblement et respectivement au diamètre intérieur et au diamètre extérieur de la fibre annulaire 32, comme on le voit sur la figure 4.

La bague 34 est faite d'une matière transparente à la lumière susceptible de se propager dans la fibre annulaire 32 et repose contre la face plane de l'extrémité de la fibre 26, en étant en coïncidence avec la fibre annulaire 32 pour constituer un prolongement optique de celle-ci, comme on le voit sur la figure 4.

La tête photo-ionique de la figure 4 comprend encore la couche mince 20 qui, dans le cas de la figure 4, repose contre la face plane de l'extrémité de la fibre centrale 30, dans l'espace délimité par la bague 34 et donc à distance de la zone 18 à traiter du matériau 10.

Le dispositif de la figure 3 comprend encore le moyen 24 de polarisation constante de la gaine conductrice 12 par rapport au matériau 10 pour établir le champ électrique mentionné plus haut dans l'espace délimité par la bague de confinement 34.

La couche 20 peut être faite d'une matière électriquement conductrice ou semiconductrice.

Cette couche 20 peut, au contraire, être faite d'une matière électriquement isolante mais, dans ce cas, on ajoute à la tête photo-ionique 8 une petite bague 36 électriquement conductrice qui est électriquement reliée à la gaine 12 et entoure la bague de confinement 34, sensiblement au niveau de cette couche isolante 20, comme on le voit sur la figure 4.

En plus du laser pulsé de puissance 4, le dispositif de la figure 3 comprend un autre laser 38 apte à émettre en continu une lumière visible pour l'observation de la zone à traiter.

Un dispositif 40 est prévu pour le couplage et l'injection optique du faisceau laser pulsé de puissance émis par le laser 4, dans la fibre 26.

Un deuxième dispositif optique 42 est également prévu pour le couplage et l'injection du faisceau laser continu d'observation émis par le laser 38, dans cette fibre 26.

Dans l'exemple représenté, les dispositifs 40 et 42 comprennent des optiques séparatrices, la lumière issue de l'optique 42 passant par l'optique 40 avant de pénétrer dans la fibre 26.

Une impulsion laser de puissance, émise par le laser 4, se propage dans la fibre centrale 30 pour interagir avec la couche 20 et permettre l'implantation d'ions dans la zone à traiter.

Dans le cas de la figure 3, la lumière visible continûment émise par le laser 38 se propage entre autres dans la fibre annulaire 32 puis dans la bague de confinement 34 (dont le matériau constitutif est transparent à cette lumière) et, lorsque cette bague n'est pas en contact avec le matériau 10, cette lumière éclaire ce matériau et retourne en partie dans la bague 34 puis dans la fibre annulaire.

Des moyens d'observation 44, comprenant par exemple un écran vidéo, sont prévus pour l'observation de la zone 18 à partir de cette lumière qui leur parvient successivement par l'intermédiaire de la fibre annulaire 32, de l'optique 40 et de l'optique 42.

On peut prévoir, comme on le voit sur les figures 2 et 4, entre la fibre optique et la couche mince 20, une autre couche mince intermédiaire 46 constituant une couche d'adaptation d'impédance optique.

Cette couche intermédiaire 46 est constituée d'un matériau permettant de réduire le saut d'indice optique entre le matériau constitutif de la fibre optique 2 ou 30, qui guide le faisceau laser de puissance pulsée, et le matériau constitutif de la couche mince 20.

La couche intermédiaire 46 permet de réduire la fraction d'énergie lumineuse réfléchie par l'interface fibre/couche mince 20 et, par conséquent, d'augmenter la densité d'énergie qui va servir à évaporer de manière explosive cette couche mince 20.

L'indice optique de la couche intermédiaire 46 est ajusté en fonction du rapport de l'indice optique de la couche mince 20 et l'indice optique du coeur de la fibre optique 2 ou 30 transmettant l'impulsion laser de puissance.

Cette adaptation des indices permet d'augmenter l'intensité de l'onde de choc induite par le plasma ionisé, d'accroître l'effet de durcissement superficiel et par conséquent l'efficacité du traitement.

La couche de matière à implanter dans la zone à traiter est consommable (elle est détruite lors du processus d'implantation ionique).

Cette matière en couche mince peut être déposée, préalablement à l'utilisation du dispositif, par un procédé de dépôt sous vide tel que le dépôt chimique en phase vapeur ("Chemical vapour deposition") ou le dépôt par plasma en phase vapeur ("Plasma vapour deposition").

Ce dépôt peut être réalisé directement sur la face plane de la fibre optique 2 ou 30 destinée à transporter le faisceau du laser de puissance.

En variante, on forme cette couche sur une pastille 48 (figure 5) de diamètre légèrement inférieur au diamètre intérieur de la bague de confinement et faite d'un matériau transparent au faisceau laser de puissance, cette pastille 48 étant ensuite optiquement couplée à la face plane de l'extrémité de la fibre 2 ou 30 transportant ce faisceau laser de puissance, comme le voit sur la figure 5.

Bien entendu, on peut prévoir la couche intermédiaire 46 entre la couche mince 20 et cette pastille 48.

On peut alors remplacer, après chaque utilisation du dispositif, l'ensemble formé par la bague de confinement et la pastille portant la couche mince 20.

Pour rendre le dispositif plus opérationnel, on peut utiliser un système à barillet (non représenté) garni de plusieurs ensembles formés chacun par la bague de confinement et la pastille 46 portant la couche mince 20 pour mettre successivement ces ensembles en regard de la face plane de l'extrémité de la fibre optique prévue pour transporter l'impulsion laser de puissance.

En variante, on réalise une bande portant la matière en couche mince à projeter et l'on fait défiler cette bande entre l'extrémité de cette fibre optique et la bague de confinement.

Ceci est plus précisément illustré par la figure 6 sur laquelle on voit cette bande 50 faite d'un matériau transparent aux faisceaux lasers utilisés et portant dans sa partie centrale une succession de cibles 20 faites du matériau en couche mince à projeter, ainsi que des moyens 52 permettant de faire défiler la bande entre l'extrémité de la fibre optique prévue pour transporter le faisceau laser de puissance et la bague de confinement, de manière à amener successivement les cibles en regard de cette fibre.

Ces moyens 52 sont montés sur la pièce destinée à rendre solidaire l'une de l'autre l'extrémité de la fibre et la bague de confinement, comme on le voit sur la figure 6.

Ainsi, lorsque le matériau en couche mince a été projeté sur une zone à traiter du matériau, on positionne la tête photo-ionique à un autre endroit à traiter du matériau et l'on fait défiler la bande 50 de manière à mettre une nouvelle couche mince en regard de la fibre optique.

La figure 7 illustre schématiquement un dispositif conforme à l'invention qui comprend des moyens permettant de positionner successivement la tête photo-ionique en des zones du matériau à traiter qui ont été déterminées à l'avance.

Le dispositif de la figure 7 comprend un bras de positionnement 54 qui est rendu solidaire de la tête photo-ionique 8.

Dans l'exemple représenté, ce bras de positionnement 54 est un bras à cinq axes (trois axes de translation et deux axes de rotation) et ce bras 54 est commandé par un robot à cinq axes 56.

Dans l'exemple représenté sur la figure 7, le dispositif comprend également des moyens permettant l'utilisation en continu de ce dispositif, comme par exemple le système à bande défilante décrit en faisant référence à la figure 6.

Le dispositif de la figure 7 comprend aussi un automate programmable 60 prévu pour commander le robot 56, le laser de puissance 4 et le laser d'observation 38 ainsi que les moyens 52 permettant le défilement de la bande portant le matériau en couche mince à projeter.

L'automate programmable 60 est commandé par un ordinateur 62 dans lequel est mémorisé un logiciel de gestion du dispositif.

Ce logiciel gère notamment les différentes opérations de mise en place de la tête photo-ionique 8 en différents endroits, repérés à l'avance, du matériau à traiter 10.

On peut adjoindre au dispositif des moyens de télémétrie laser (non représentés), permettant de mesurer la distance de la tête photo-ionique au matériau à traiter.

On peut également adjoindre au dispositif de la figure 7 une caméra (non représentée) permettant de visualiser l'ensemble du matériau ou de la pièce à traiter et de déterminer la position de la tête photo-ionique par rapport à cette pièce, pour pouvoir positionner convenablement cette tête en regard de la zone que l'on veut traiter.

La figure 8 illustre schématiquement et partiellement un autre dispositif conforme à l'invention, dans lequel la tête photo-ionique comprend une diode ou micropuce laser 64 sur laquelle est formée la couche mince 20.

Les bornes de cette diode laser 64 sont respectivement reliées à la gaine conductrice 12 et à la couche mince 20.

Comme on le voit sur la figure 8, cette diode laser 64 est placée contre la face plane de la fibre optique destinée à transporter le faisceau laser de puissance, en interposant éventuellement la couche intermédiaire 46 entre cette fibre optique et la diode laser (qui est logée dans l'espace délimité par la bague de confinement 16 ou 34).

Avec cette diode laser, on peut utiliser le laser de puissance 4 pour le pompage optique de cette diode laser qui est polarisée grâce au moyen de polarisation 24 (la cible 20 jouant le rôle de masse).

Cependant, au lieu du laser 4, on peut utiliser une source lumineuse 66 (figure 1) apte à émettre une lumière pulsée incohérente de grande intensité (par exemple une lampe flash), ce qui permet également le pompage optique de la diode laser 64.

La figure 9 illustre schématiquement une variante de réalisation du dispositif objet de l'invention, dans laquelle la couche mince 20 est formée sur une face convexe 67 d'une lentille convexe 68 qui est optiquement couplée par son autre face 69 à l'extrémité de la fibre optique destinée à transporter le faisceau laser pulsé de puissance.

La face 69 de la lentille peut être plane ou convexe (cas de la figure 9), auquel cas l'extrémité de la fibre à laquelle elle est couplée présente une concavité adaptée à cette face 69.

On voit sur la figure 9 la face convexe 67 portant la couche mince 20 et se trouvant dans l'espace délimité par la bague de confinement, à distance du matériau à traiter, cette lentille biconvexe 68 permettant la focalisation, sur la couche mince, des impulsions lasers de puissance qui lui parviennent.

Une variante de réalisation est schématiquement illustrée par la figure 10 où cette lentille est supprimée et l'extrémité 72 de la fibre optique prévue pour transporter le faisceau laser de puissance a une forme convexe et la couche mince 20 est formée sur cette face convexe, cette convexité permettant encore la focalisation, sur cette couche mince, du faisceau laser de puissance.

La figure 11 illustre schématiquement et partiellement un autre dispositif conforme à l'invention, dans lequel la bague de confinement n'est munie d'aucune couche mince de matériau à projeter.

L'espace délimité par cette bague de confinement est rempli d'un fluide 74 (gaz ou liquide), qui est maintenu par capillarité dans cet espace (dans le cas d'un liquide), ce liquide ou ce gaz étant transparent au faisceau laser de puissance, ou photodécomposable et phototionisable sous l'effet de ce faisceau laser.

Le dispositif schématiquement illustré par la figure 11 permet le durcissement, par confinement de plasma de surface, du matériau à traiter sans apport de matière dans le cas d'un fluide transparent.

Dans le cas où le fluide est photodécomposable et photoionisable, le durcissement se fait par les espèces chimiques produites lors du processus de photoionisation et photodécomposition.

Dans le cas de la figure 11, la gaine conductrice 12 peut être supprimée et la gaine isolante protectrice 14 est alors directement en contact avec la fibre optique annulaire 32 (ou, dans un dispositif sans moyens d'observation de la zone à traiter, en contact avec la fibre optique 2).

Dans le cas de la figure 11, le moyen 24 de polarisation peut ne pas être utilisé.

La figure 12 illustre schématiquement un dispositif conforme à l'invention, comprenant ne pluralité de fibres optiques 2 ou 26 respectivement munies de têtes photo-ioniques 8, en vue de traiter simultanément différentes zones de la pièce 10 au moyen du même laser de puissance 4, dont le faisceau pulsé est simultanément injecté dans les différentes fibres optiques.

Bien entendu, on peut utiliser un robot pour positionner respectivement ces têtes photo-ioniques au niveau des zones correspondantes.

On peut réaliser un dispositif conforme à l'invention permettant de visualiser une pièce de forme quelconque sur un moniteur vidéo, de repérer la ou les zones à traiter choisies, de placer la ou les têtes photo-ioniques en cette ou ces zones et de traiter ces différentes zones pour traiter ensuite, si nécessaire, d'autres zones en déplaçant convenablement les têtes photo-ioniques et en disposant de nouvelles couches minces à projeter dans ces têtes photo-ioniques (par exemple avec un dispositif à fonctionnement en continu comme on l'a vu plus haut dans un exemple).

Avec un dispositif conforme à l'invention, on remédie à l'inconvénient du dispositif connu décrit dans le document (2) où il est nécessaire de corréler spatialement le laser de puissance et la cellule d'implantation ionique assistée par laser.

Au contraire, dans un dispositif conforme à l'invention, la corrélation spatiale entre le laser de puissance et la tête photo-ionique n'est pas nécessaire.

Dans l'invention, l'ensemble comprenant le laser de puissance (où la source incohérente de puissance et la diode laser), la fibre optique et la tête photo-ionique peut être aisément rendu compact, scellé et étanche à l'atmosphère extérieur, par exemple en utilisant un tuyau flexible dans lequel on fait le vide et l'on fait passer la fibre optique, en reliant de façon étanche une extrémité de ce tuyau au laser et l'autre extrémité à la bague de confinement.

Ceci permet d'utiliser un dispositif conforme à l'invention en atmosphère radioactive, sous la mer ou même dans l'espace.

## Revendications

1. Dispositif de traitement d'un matériau, caractérisé en ce qu'il comprend :
- au moins une fibre optique (2, 26),
- une première source de lumière (4, 66) prévue pour engendrer une lumière pulsée permettant le traitement du matériau (10),
- un moyen d'injection (6, 40) de cette lumière pulsée dans une première extrémité de chaque fibre optique, et
- pour chaque fibre optique, une tête photo-ionique miniaturisée (8) dont les dimensions transversales ne dépassent que d'environ 20% celles de la fibre optique et comprenant une bague de confinement (16, 34) dont un premier côté est rendu solidaire de la deuxième extrémité de la fibre tandis que le deuxième côté de la bague est destiné à être placé contre le matériau (10), cette bague étant apte à confiner un plasma susceptible de se former, grâce à la lumière pulsée, dans l'espace (19) délimité par cette bague, à partir du matériau (10) ou d'une matière (20) qui est placée dans cet espace, en vue du traitement du matériau.

2. Dispositif selon la revendication 1, caractérisé en ce que la bague de confinement (16, 34) est électriquement isolante, en ce que chaque fibre optique (2, 26) est revêtue d'une gaine électriquement conductrice (12), en ce que la matière placée dans l'espace délimité par la bague de confinement forme une couche mince solide (20) qui est placée contre la deuxième extrémité de la fibre optique et se trouve à distance du matériau (10) et qui est destinée à se transformer, grâce à la lumière pulsée, en un plasma contenant des ions, et en ce que le dispositif comprend en outre un moyen de polarisation (24) de la gaine électriquement conductrice (12) par rapport au matériau pour créer, dans l'espace délimité par la bague de confinement, un champ électrique apte à accélérer les ions du plasma vers le matériau en vue de les implanter dans celui-ci.

3. Dispositif selon la revendication 2, caractérisé en ce que la matière formant la couche mince solide (20) est électriquement conductrice ou semiconductrice.

4. Dispositif selon la revendication 2, caractérisé en ce que la matière formant la couche mince solide (20) est électriquement isolante et en ce que le dispositif comprend en outre une bague (36) qui est électriquement conductrice et reliée à la gaine conductrice (12) et qui est placée sensiblement au niveau de la couche mince (20), contre la paroi externe de la bague de confinement (16, 34).

5. Dispositif selon l'une quelconque des revendications 2 à 4, caractérisé en ce qu'il comprend en outre une lentille (68) qui est optiquement couplée à la deuxième extrémité de la fibre optique et qui comporte une face convexe (67) dans l'espace délimité par la bague de confinement (16, 34), cette face étant destinée à se trouver du côté du matériau à traiter, et en ce que la couche mince (20) est formée sur cette face convexe (67).

6. Dispositif selon l'une quelconque des revendications 2 à 4, caractérisé en ce que la deuxième extrémité de la fibre optique comporte une partie convexe (72) dans l'espace délimité par la bague de confinement et en ce que la couche mince (20) est formée sur cette partie convexe (72).

7. Dispositif selon l'une quelconque des revendications 2 à 4, caractérisé en ce qu'il comprend en outre une pastille (48) qui est transparente à la lumière pulsée, qui est optiquement couplée à la deuxième extrémité de la fibre optique (2, 26) et placée dans l'espace délimité par la bague de confinement (16, 34) et dont la face destinée à se trouver du côté du matériau à traiter porte la couche mince (20).

8. Dispositif selon l'une quelconque des revendications 2 à 4, caractérisé en ce qu'il comprend en outre :
- une bande (50) sur laquelle est formée la couche mince (20), et
- un moyen (52) de défilement de cette bande entre la deuxième extrémité de la fibre optique et la bague de confinement.

9. Dispositif selon l'une quelconque des revendications 2 à 4, caractérisé en ce qu'il comprend en outre une couche intermédiaire (46) entre la fibre et la couche mince (20), cette couche intermédiaire étant prévue pour adapter l'indice optique de la fibre à celui de la couche mince, pour réduire la quantité de lumière pulsée susceptible d'être réfléchie par cette couche mince.

10. Dispositif selon la revendication 1, caractérisé en ce qu'il comprend en outre, dans l'espace délimité par la bague de confinement (16, 34), un fluide (74) qui remplit cet espace et qui est transparent à la lumière pulsée, de façon à durcir le matériau par confinement du plasma formé par interaction de la lumière pulsée avec ce matériau, ou qui est photodécomposable et photoionisable par cette lumière pulsée, de façon à traiter chimiquement la surface du matériau par les espèces chimiques produites lors du processus de photoionisation et photodécomposition.

11. Dispositif selon l'une quelconque des revendications 1 à 10, caractérisé en ce que la première source de lumière est un laser de puissance pulsé (4).

12. Dispositif selon l'une quelconque des revendications 1 à 10, caractérisé en ce que la première source de lumière est une source de puissance (66), de lumière incohérente pulsée, et en ce que le dispositif comprend en outre une diode-laser (64) qui est optiquement couplée à la deuxième extrémité de la fibre optique (2, 26) et qui est apte à engendrer un faisceau laser pulsé de puissance lorsqu'elle reçoit la lumière pulsée de la première source (66).

13. Dispositif selon l'une quelconque des revendications 1 à 12, caractérisé en ce que la fibre optique (26) comprend :
- une fibre optique centrale (30), destinée à transmettre la lumière pulsée, et
- une fibre optique annulaire (32) qui entoure cette fibre optique centrale,
et en ce que le dispositif comprend en outre :
- une deuxième source de lumière (38) prévue pour engendrer une lumière visible permettant l'observation du matériau (10),
- un moyen d'injection (42) de cette lumière d'observation dans la fibre optique annulaire (32), et
- un moyen (44) de visualisation de l'image du matériau, à partir de la lumière d'observation réfléchie par le matériau et transmise par la fibre optique annulaire (32).

14. Dispositif selon l'une quelconque des revendications 1 à 13, caractérisé en ce qu'il comprend en outre un moyen (54, 56) de positionnement de la tête optique (8) en regard d'une zone choisie du matériau.

15. Dispositif selon l'une quelconque des revendications 1 à 14, caractérisé en ce qu'il comprend une pluralité de fibres optiques (2, 26) respectivement associées à une pluralité de têtes photo-ioniques (8), pour traiter simultanément une pluralité de zones du matériau (10).

## Patentansprüche

1. Vorrichtung zur Materialbearbeitung, dadurch gekennzeichnet, daß sie umfaßt:
- wenigstens eine optische Faser (2, 26),
- eine erste Lichtquelle (4, 66), vorgesehen zum Erzeugen eines pulsierenden bzw. gepulsten Lichts in einem ersten Ende jeder optischen Faser, und
- für jede optische Faser einen miniaturisierten photoionischen Kopf (8), dessen Querabmessungen diejenigen der optischen Faser um nicht mehr als ungefähr 20% überschreiten und die einen Einschließungsring (16, 34) umfassen, von dem eine erste Seite fest verbunden ist mit dem zweiten Ende der Faser, während die zweite Seite des Rings dazu bestimmt ist, an dem Material (10) angebracht zu werden, wobei dieser Ring ein Plasma einschließen kann, das sich dank des gepulsten Lichts in dem durch diesen Ring abgegrenzten Raum (19) aus dem Material (10) oder einem Material (20) bilden kann, das sich in diesem Raum befindet, im Hinblick auf die Bearbeitung des Materials.

2. Vorrichtung nach Anspruch 1, dadurch gekennzeichnet, daß der Einschließungsring (16, 34) elektrisch isolierend ist, daß jede optische Faser (2, 26) mit einer elektrisch leitenden Hülle (12) überzogen ist, daß das in dem durch den Einschließungsring abgegrenzten Raum befindliche Material eine feste Dünnschicht (20) bildet, die am zweiten Ende der optischen Faser angebracht und vom Material (10) beabstandet ist und die dazu bestimmt ist, sich dank des gepulsten Lichts in ein Plasma zu verwandeln, das Ionen enthält, und dadurch, daß die Vorrichtung außerdem eine Einrichtung (24) zum Polarisieren bzw. Vorspannen der elektrisch leitenden Hülle (12) in bezug auf das Material umfaßt, um in dem durch den Einschließungsring abgegrenzten Raum ein elektrisches Feld zu erzeugen, das fähig ist, die Ionen des Plasmas zu Richtung Material zu beschleunigen, um sie in dieses zu implantieren.

3. Vorrichtung nach Anspruch 2, dadurch gekennzeichnet, daß das die feste Dünnschicht bildende Material (20) elektrisch leitend oder halbleitend ist.

4. Vorrichtung nach Anspruch 2, dadurch gekennzeichnet, daß das die feste Dünnschicht (20) bildende Material elektrisch isolierend ist und daß die Vorrichtung außerdem einen Ring (36) umfaßt, der elektrisch leitend und verbunden ist mit der leitenden Hülle (12) und der im wesentlichen in Höhe der Dünnschicht (20) angeordnet ist, an der Außenwand des Einschließungsrings (16, 34).

5. Vorrichtung nach einem der Ansprüche 2 bis 4, dadurch gekennzeichnet, daß sie außerdem eine Linse (68) umfaßt, die optisch gekoppelt ist mit dem zweiten Ende der optischen Faser und die in dem durch den Einschließungsring (16, 34) begrenzten Raum eine konvexe Seite (67) aufweist, wobei diese Seite sich auf der Seite des zu bearbeitenden Materials befindet, und dadurch, daß die Dünnschicht (20) auf dieser konvexen Seite (67) gebildet ist.

6. Vorrichtung nach einem der Ansprüche 2 bis 4, dadurch gekennzeichnet, daß das zweite Ende der optischen Faser in dem durch den Einschließungsring abgegrenzten Raum einen konvexen Teil (72) umfassen, und dadurch, daß die Dünnschicht (20) auf diesem konvexen Teil (72) gebildet ist.

7. Vorrichtung nach einem der Ansprüche 2 bis 4, dadurch gekennzeichnet, daß sie außerdem ein Plättchen (48) umfaßt, durchlässig für das gepulste Licht und optisch gekoppelt mit dem zweiten Ende der optischen Faser (2, 26), das in dem durch den Einschließungsring (16, 34) abgegrenzten Raum angeordnet ist und dessen dem zu bearbeitenden Material zugewandte Seite die Dünnschicht (20) trägt.

8. Vorrichtung nach einem der Ansprüche 2 bis 4, dadurch gekennzeichnet, daß sie außerdem umfaßt:
- ein Band bzw. einen Streifen (50), auf dem die Dünnschicht (20) ausgebildet ist, und
- eine Einrichtung (52), um diesen Streifen zwischen dem zweiten Ende der optischen Faser und dem Einschließungsring durchlaufen zu lassen.

9. Vorrichtung nach einem der Ansprüche 2 bis 4, dadurch gekennzeichnet, daß sie außerdem eine Zwischenschicht (46) zwischen der Faser und der Dünnschicht (20) umfaßt, wobei diese Zwischenschicht vorgesehen ist, um den optischen Index der Faser an den der Dünnschicht anzupassen, um die durch diese Dünnschicht reflektierte Pulslichtmenge zu reduzieren.

10. Vorrichtung nach Anspruch 1, dadurch gekennzeichnet, daß sie außerdem in dem durch den Einschließungsring (16, 34) begrenzten Raum ein Fluid (74) enthält, das diesen Raum füllt und durchlässig ist für das gepulste Licht, um das Material zu härten durch Einschließung des Plasmas, gebildet durch Wechselwirkung des gepulsten Lichts mit diesem Material, oder das photozersetzbar oder photoionisierbar ist durch dieses gepulste Licht, um die Materialoberfläche durch die bei dem Prozeß der Photoionisation und der Photozersetzung erzeugten Arten (espèces) chemisch zu behandeln.

11. Vorrichtung nach einem der Ansprüche 1 bis 10, dadurch gekennzeichnet, daß die erste Lichtquelle ein leistungsstarker Puls- bzw. Impulslaser (4) ist.

12. Vorrichtung nach einem der Ansprüche 1 bis 10, dadurch gekennzeichnet, daß die erste Lichtquelle eine Leistungsquelle (66) mit gepulstem inkohärentem Licht ist, und dadurch, daß die Vorrichtung außerdem eine Laserdiode (64) umfaßt, die optisch mit dem zweiten Ende der optischen Faser (2, 26) gekoppelt ist und die fähig ist, einen leistungsstarken pulsierenden bzw. gepulsten Laserstrahl zu erzeugen, wenn sie das gepulste Licht der ersten Quelle (66) empfängt.

13. Vorrichtung nach einem der Ansprüche 1 bis 12, dadurch gekennzeichnet, daß die optische Faser umfaßt:
- eine zentrale optische Faser (30) zum übertragen des gepulsten Lichts, und
- eine ringförmige optische Faser (32), die diese zentrale optische Faser umgibt,
und dadurch, daß die Vorrichtung außerdem umfaßt:
- eine zweite Lichtquelle (38) zum Erzeugen eines sichtbaren Lichts, das die Beobachtung des Materials (10) ermöglicht,
- eine Einrichtung (42) zum Einspeisen dieses Beobachtungslichts in die ringförmige optische Faser (32), und
- eine Vorrichtung (44) zum Sichtbarmachen der Abbildung des Materials aufgrund des Lichts, das durch das Material reflektiert wird und durch die ringförmige optische Faser (32) übertragen wird.

14. Vorrichtung nach einem der Ansprüche 1 bis 13, dadurch gekennzeichnet, daß sie außerdem eine Einrichtung (54, 56) zum Positionieren des optischen Kopfes (8) gegenüber einer ausgewählten Material zone umfaßt.

15. Vorrichtung nach einem der Ansprüche 1 bis 14, dadurch gekennzeichnet, daß sie eine Vielzahl optischer Fasern (2, 26) umfaßt, jeweils verbunden mit einer Vielzahl photoionischer Köpfe (8), um simultan eine Vielzahl von Zonen des Materials (10) zu bearbeiten.

## Claims

1. Apparatus for the treatment of a material, characterized in that it comprises:
- at least one optical fibre (2, 26),
- a first light source (4, 66) for producing a pulsed light permitting the treatment of the material (10),
- a means (6, 40) for injecting said pulsed light into a first end of each optical fibre and
- for each optical fibre, a miniaturized photoionic head (8), whose transverse dimensions only exceed by approximately 20% those of the optical fibre and having a confinement ring (16, 34), whereof a first side is joined to the second end of the fibre, whereas the second side of the ring is to be placed against the material (10), said ring being able to confine a plasma which can form, due to the pulsed light, in the space (19) defined by said ring, from the material (10) or substance (20) placed in said space, with a view to the treatment of the material.

2. Apparatus according to claim 1, characterized in that the confinement ring (16, 34) is electrically insulating, each optical fibre (2, 26) being coated with an electrically conductive sheath (12), the material placed in the space defined by the confinement ring forms a solid thin film (20) placed against the second end of the optical fibre and at a distance from the material (10) and which is to be transformed, by means of the pulsed light, into a plasma containing ions, and the apparatus also comprises a means (24) for polarizing the electrically conductive sheath (12) with respect to the material in order to create, in the space defined by the confinement ring, an electrical field able to accelerate the ions of the plasma towards the material with a view to implanting them therein.

3. Apparatus according to claim 2, characterized in that the substance forming the thin, solid film (20) is electrically conductive or semiconducting.

4. Apparatus according to claim 2, characterized in that the substance forming the thin, solid film (20) is electrically insulating and in that the apparatus also comprises a ring (36), which is electrically conducting
and connected to the conductive sheath (12) and which is placed substantially level with the thin film (20), against the outer wall of the confinement ring (16, 34).

5. Apparatus according to any one of the claims 2 to 4, characterized in that it also comprises a lens (68), which is optically coupled to the second end of the optical fibre and which has a convex face (67) in the space defined by the confinement ring (16, 34), said face is to be located on the side of the material to be treated and in that the thin film (20) is formed on said convex face (67).

6. Apparatus according to any one of the claims 2 to 4, characterized in that the second end of the optical fibre has a convex portion (72) in the space defined by the confinement ring and in that the thin film (20) is formed on said convex portion (72).

7. Apparatus according to any one of the claims 2 to 4, characterized in that it also comprises a pellet (48), which is transparent to the pulsed light, which is optically coupled to the second end of the optical fibre (2, 26) and which is placed in the space defined by the confinement ring (16, 34) and whose face which is to be on the side of the material to be treated carries the thin film (20).

8. Apparatus according to any one of the claims 2 to 4, characterized in that it also comprises a strip (50) on which is formed the thin film (20) and a means (52) for moving said strip between the second end of the optical fibre and the confinement ring.

9. Apparatus according to any one of the claims 2 to 4, characterized in that it also comprises an intermediate film (46) between the fibre and the thin film (20), said intermediate film serving to adapt the optical index of the fibre to that of the thin film, so as to reduce the pulsed light quantity liable to be reflected by said thin film.

10. Apparatus according to claim 1, characterized in that it also comprises, in the space defined by the confinement ring (16, 34), a fluid (74) filling said space and which is transparent to the pulsed light, so as to harden the material by the confinement of the plasma formed by the interaction of the pulsed light with said material, or which is photodecomposable and photoionizable by said pulsed light, so as to chemically treat the surface of the material by chemical species produced during the photoionization and photodecomposition process.

11. Apparatus according to any one of the claims 1 to 10, characterized in that the first light source is a pulsed power laser (4).

12. Apparatus according to any one of the claims 1 to 10, characterized in that the first light source is a pulsed, incoherent power light source (66) and in that the apparatus also comprises a laser diode (64) optically coupled to the second end of the optical fibre (2, 26) and able to produce a pulsed power laser beam when it receives pulsed light from the first source (66).

13. Apparatus according to any one of the claims 1 to 12, characterized in that the optical fibre (26) comprises:
- a central optical fibre (30) for transmitting the pulsed light and
- an annular optical fibre (32) surrounding the central optical fibre, and the apparatus also comprises:
- a second light source (38) for producing a visible light permitting the observation of the material (10),
- a means (42) for injecting said observation light into the annular optical fibre (32) and
- a means (44) for displaying the image of the material, from the observation light reflected by the material and transmitted by the annular optical fibre (32).

14. Apparatus according to any one of the claims 1 to 13, characterized in that it also comprises a means (54, 56) for positioning the optical head (8) in front of a chosen area of the material.

15. Apparatus according to any one of the claims 1 to 14, characterized in that it comprises a plurality of optical fibres (2, 26), respectively associated with a plurality of photoionic heads (8), in order to simultaneously treat a plurality of areas of the material (10).
